# EUROPÄISCHE PATENTSCHRIFT

(11) **EP 2 198 779 B1**
(45) Veröffentlichungstag und Bekanntmachung des Hinweises auf die Patenterteilung: **19.09.2018**
(21) Anmeldenummer: 08022336.5
(22) Anmeldetag: 22.12.2008
(51) Int. Cl.: A61B 5/08, A61B 5/087, A61B 5/091, A61B 5/11, A61B 5/22

(54) **Vorrichtung und Verfahren zur Früherkennung von Exazerbationen**
Device and method for early detection of exacerbations
Dispositif et procédé destinés à la détection précoce d'exacerbations

(43) Veröffentlichungstag der Anmeldung: 23.06.2010
(73) Patentinhaber: Sendsor GmbH, 80335 München (DE)
(72) Erfinder: Scholz, Alexander, Dipl.-Ing., 83483 Bischofswiesen (DE); Kenn, Klaus, Dr. med., 83483 Bischofswiesen (DE); Gül, Murat, Dipl.-Ing., 80801 München (DE); Dill, Dieter, Dipl.-Ing., 83670 Bad Heilbrunn (DE)
(74) Vertreter: Müller Hoffmann & Partner

(56) Entgegenhaltungen:
- EP-A- 1 588 662
- WO-A-01/52718
- WO-A-2005/037077
- US-A1- 2001 049 470
- US-A1- 2004 249 299
- US-A1- 2005 080 348
- US-B1- 6 183 423

## Beschreibung

Die Erfindung betrifft eine Vorrichtung zum Erfassen von Parametern, welche für eine Diagnose einer Exazerbation im Rahmen einer Lungenerkrankung eines Probanden von Bedeutung sein können, mit einer Auswerteeinheit zum Sammeln von Messdaten aus einer Spirometereinheit und einer Aktivitätsmesseinheit der Vorrichtung.

Unter einer Exazerbation wird ein Ausbruch eines Krankheitsschubs bei chronisch verlaufenden Erkrankungen verstanden, wobei der genaue Zeitpunkt einer Exazerbation in der Regel nicht vorhersagbar ist. Im Rahmen einer chronischen Lungenerkrankung kann sich eine Exazerbation beispielsweise in einem rapiden Abfallen der Lungenleistung eines Patienten äußern, sodass dieser beispielsweise nur sehr schwach und flach atmen kann. Derartige Exazerbationen bei einer schweren chronischen Lungenerkrankung sind oft lebensbedrohlich und gehen zudem oft mit Angstzuständen des Patienten einher. Die Gründe für eine Exazerbation, insbesondere bei deren plötzlichem Auftreten können sich direkt aus der Expression der entsprechenden Krankheit ergeben oder indirekt, beispielsweise durch eine allgemeine Schwächung von durch die Krankheit betroffenen Körperregionen oder des gesamten Immunsystems erfolgen. Bei einer chronisch obstruktiven Lungenerkrankung (engl. chronic obstructive pulmonary disease, COPD) können sich durch die allgemeine Verschlechterung des Zustands der Lunge Viren und Bakterien darin ausbreiten. Eine daraus folgende Infektion führt meist zu einer erkennbaren Verfärbung des Auswurfs (Sputum) sowie zu starkem Husten und akuter Luftnot und wird auch als "Infektexazerbation" bezeichnet. Bei einer COPD treten Exazerbationen bisher meist völlig unvorhersagbar auf, sodass die Lebensqualität von Patienten neben dem Krankheitsbild selbst auch darunter leidet, dass jederzeit mit einer Exazerbation gerechnet werden muss.

Insbesondere im Rahmen einer COPD, aber auch bei einer Vielzahl weiterer Lungenerkrankungen kann bei einer bereits diagnostizierten Krankheit eine zeitliche Abschätzung eines Auftretens einer Exazerbation die Lebensqualität von betroffenen Patienten erheblich steigern und eine Möglichkeit bieten, früh genug Gegenmaßnahmen gegen eine Exazerbation bzw. deren Symptome einzuleiten oder bereitzustellen.

Aus EP 1 588 662 A ist ein Biosensor zum Messen von kardiopulmonarer Aktivität bekannt. Ein entsprechendes tragbares Sensorgerät soll eine Volumenmessung der Lunge eines Probanden erlauben. Ferner wird die Lungenaktivität mittels eines Sensors ermittelt, wobei kein Spirometer verwendet werden soll.

Aus US 2004/249299 A1 sind Verfahren und Systeme zur Analyse von physiologischen Signalen bekannt. Es sollen oximetrische Signale, sowie Herzsignale und Lungensignale neben Körperlagesignalen erfasst werden und in einer gemeinsamen Signalverarbeitung weiterverarbeitet werden. Anhand dieser Signale soll eine physiologische Erkennung des zugrundeliegenden Objekts möglich sein.

Aus US 2005/080348 A1 sind ein System und ein Verfahren zum Nachvollziehen medizinischer Eingriffe bekannt. Hierbei sollen Messungen des Herzschlagverhaltens eines Patienten durchgeführt werden. Ferner sollen medizinische Daten über das Atemverhalten des entsprechenden Patienten erfasst werden.

US2001/0049470 offenbart eine Aktivitätsmessgerät zur Unterstützung einer Diät, welches sowohl eine Geschwindingkeits-oder Beschleunigungseinheit und eine Spirometereinheit umfasst.

In US 6,183,423 B1 sind eine Vorrichtung und ein Verfahren zum Überwachen einer Unregelmäßigkeit des Atemvorgangs eines Patienten bekannt. Ein entsprechendes Gerät soll zur Diagnose von Fehlverhalten in der Atemfunktion eines Patienten dienen.

Der Erfindung liegt die Aufgabe zugrunde, eine Vorrichtung zum Erfassen von Exazerbations-relevanten Parametern im Rahmen einer Lungenerkrankung eines Probanden anzugeben. Außerdem liegt der Erfindung die Aufgabe zugrunde, ein Verfahren zum Erfassen und Vergleichen von Exazerbations-relevanten Parametern eines Probanden anzugeben.

Erfindungsgemäß wird die Aufgabe durch eine Vorrichtung gemäß Patentanspruch 1 gelöst. Ein entsprechendes Verfahren ist in Anspruch 13 definiert. Vorteilhafte Weiterentwicklungen der Erfindung sind in den abhängigen Ansprüchen angegeben.

Eine erfindungsgemäße Vorrichtung dient zum Erfassen von Exazerbations-relevanten Parametern im Rahmen einer Lungenerkrankung eines Probanden, insbesondere eines menschlichen Patienten, und weist eine Spirometereinheit, eine Aktivitätsmesseinheit sowie eine Auswerteeinheit auf. Dabei dient die Spirometereinheit zum Messen der Lungenatmung des Probanden, die Aktivitätsmesseinheit zum Messen einer motorischen Bewegungsaktivität des Probanden und die Auswerteeinheit zum Sammeln von Messdaten der Spirometereinheit und der Aktivitätsmesseinheit.

Die Spirometereinheit zum Messen der Lungenatmung des Probanden kann Atemluftvolumina bei Ein- und/oder Ausatemvorgängen des Probanden, also sein Ein- oder Ausatmemvolumen (sog. inspiratorische bzw. exspiratorische Volumina), sein gesamtes Lungenvolumen, den zeitlichen Verlauf eines Ein- oder Ausatemdruckes, den Minimal- und/oder Maximalwert eines Volumen- oder Masseflusses bei der Atmung des Probanden, sowie eine entsprechende Flussgeschwindigkeit, einen Massefluss oder einen Volumenfluss von Atemvorgängen über einen Zeitraum messen. Demnach weist die Spirometereinheit die Funktionen eines Spirometers und eines Pneumotachographen auf.

Die Spirometereinheit weist zum Messen der oben genannten Parameter wie Flussgeschwindigkeiten, Volumina oder Drücke beispielsweise einen Volumensensor, einen Volumenflusssensor, einen Massesensor, einen Masseflusssensor, einen Drucksensor, einen Temperatursensor und/oder einen Feuchtigkeitssensor auf. Es können selbstverständlich auch mehrere Sensoren vom gleichen Typ eingesetzt werden, also zum Beispiel mehrere Drucksensoren. Für die Messung eines Parameters können überdies ein Sensor eines Sensortyps oder mehrere Sensoren unterschiedlicher Typen verwendet werden. Beispielsweise kann ein Volumenfluss einer in einem Ausatemvorgang eines Probanden ausgeatmeten Atemluft mithilfe eines Volumenflusssensors gemessen werden. Ein Volumenfluss kann jedoch auch mithilfe eines Masseflusssensors, eines Temperatursensors und eines Feuchtigkeitssensors gemessen werden, indem der entsprechende Massefluss, die Temperatur und die Luftfeuchtigkeit der ausgeatmeten Atemluft gemessen werden und daraus ein Messwert für den Volumenfluss bestimmt wird. Eine derartige Bestimmung kann beispielsweise auf festgelegten Auswertealgorithmen direkt gemessener Parameter beruhen und stellt somit im engeren Sinne keine reine Messung dar. Jedoch sei eine indirekte Bestimmung des Volumenflusses durch eine Berechnung von Messwerten, ebenso wie aller weiteren genannten Parameter hier dennoch als Messung durch die Spirometereinheit verstanden. Entscheidend ist hier, dass die genannten Daten über die Lungenfunktion eines Probanden basierend auf Messungen durch die Spirometereinheit ermittelbar sind und für weitere Einheiten der Vorrichtung, insbesondere die Aktivitätsmesseinheit und die Auswerteeinheit bereitstellbar sind.

Die oben aufgezählten Messgrößen der Lungenatmung eines Probanden können für eine Exazerbation im Rahmen einer Lungenerkrankung von Bedeutung sein und ändern sich in der Regel bei einem Auftreten bzw. während einer Exazerbation. Allerdings kann ein Zeitpunkt einer Exazerbation im Rahmen einer Lungenerkrankung in der Regel nicht mit einem Spirometer oder einem Pneumotachographen prognostiziert werden, da sich die entsprechenden Messgrößen erst beim tatsächlichen Auftreten der Exazerbation oder erst unmittelbar davor verändern, nicht aber bereits im Vorfeld einer Exazerbation. Bei einer Betrachtung der Lungenatmung und zusätzlichen Parametern am Probanden, die nicht direkt mit der Lungenatmung zusammenhängen, ist jedoch eine zu einer Prognose über einen Zeitpunkt eines Auftretens einer Exazerbation aufgrund einer Diagnose möglich, die mithilfe von durch die erfindungsgemäße Vorrichtung ermittelten Daten erstellbar ist.

Mit der Aktivitätsmesseinheit der Vorrichtung ist eine Kraftausübung, eine Beschleunigung, eine Zeitdauer, eine Richtung, ein Weg bzw. eine Wegstrecke, eine Geschwindigkeit, eine Häufigkeit, ein Drehimpuls und/oder ein Drehmoment einer Bewegung der Aktivitätsmesseinheit und/oder des Probanden messbar. Das Bestimmen dieser physikalischen Parameter erlaubt ein Messen einer motorischen Bewegungsaktivität des Probanden durch die Aktivitätsmesseinheit, also beispielsweise ein Messen einer aktiven oder passiven Bewegungsstärke bzw. Bewegungshäufigkeit des Probanden. Somit ist beispielsweise bestimmbar, wie stark und wie häufig sich der Proband bewegt oder bewegen lässt. Mit der Aktivitätsmesseinheit ist somit beispielsweise messbar, ob der Proband viel Sport treibt, viel zu Fuß geht oder sich aber kaum bewegt und nur mit dem Auto fährt. Es ist vorzugsweise auch messbar, wie stark der Proband seine Arme oder seine Beine bewegt, also inwieweit sich seine Extremitäten bzw. sein Rumpf zueinander bewegen. Dies kann ausschließen, dass eine passive Bewegung des Probanden beispielsweise durch eine Busfahrt, bei der sich der Patient tatsächlich motorisch im Wesentlichen nicht aktiv bewegt, mit einer tatsächlichen Bewegungsaktivität, beispielsweise im Rahmen eines Treppensteigens oder bei der Hausarbeit des Probanden gleichgesetzt bzw. verwechselt wird, sodass stets die tatsächliche motorische Aktivität des Probanden messbar ist. Demnach sei hier unter einer motorischen Bewegungsaktivität des Probanden seine tatsächliche körperliche Aktivität bzw. das Bewegen seiner Extremitäten und seines Rumpfes verstanden.

Die Aktivitätsmesseinheit kann dafür ausgelegt sein, direkt am Probanden getragen zu werden. In diesem Fall entspricht beispielsweise eine Beschleunigung der Aktivitätsmesseinheit auch einer Beschleunigung des Probanden. Jedoch ist es auch möglich, dass die Aktivitätsmesseinheit die motorische Bewegungsaktivität des Probanden aus der Ferne messen kann, so dass sie nicht am Probanden getragen werden soll.

Zum Messen der motorischen Bewegungsaktivität des Probanden kann die Aktivitätsmesseinheit beispielsweise einen Massenträgheitssensor, also einen auf dem Ausnutzen einer Massenträgheit basierenden Bewegungs- oder Beschleunigungssensor, ein Gyroskop, einen Bewegungssensor, einen Beschleunigungssensor, einen Geschwindigkeitssensor, einen Laserkreisel oder einen Faserkreisel aufweisen. Mit einem Gyroskop der Aktivitätsmesseinheit lassen sich beispielsweise Drehungen oder Neigungen sensorisieren. Ein Massenträgheitssensor der Aktivitätsmesseinheit kann beispielsweise ein kapazitives MEMS (engl. micro electromechanical system, mikro-elektromecahnisches System)-Element mit einer Beschleunigung des Elements entsprechend veränderbaren Kapazität. Ein Bewegungssensor der Aktivitätsmesseinheit kann beispielsweise eine lineare oder nichtlineare Bewegung in eine oder mehrere Richtungen sensorisieren oder beispielsweise wie ein Bewegungsmelder ein Vorhandensein oder eine Bewegung eines Objektes in einem Messbereich sensorisieren. Hierbei kann die Aktivitätsmesseinheit für eine Messung der motorischen Bewegungsaktivität sowohl am Probanden getragen werden als auch die Bewegungen des Probanden aus der Ferne sensorisieren.

Die oben beispielhaft aufgezählten Sensoren der Aktivitätsmesseinheit können auch miteinander kombiniert werden, erlauben aber in jedem Fall eine Messung einer motorischen Bewegungsaktivität des Probanden. Das bedeutet, dass beispielsweise der Geschwindigkeitssensor die Geschwindigkeit der Aktivitätsmesseinheit sensorisiert, falls diese dafür bestimmt ist, direkt am Probanden getragen zu werden. Denn in diesem Fall entspricht die Bewegungsgeschwindigkeit der Aktivitätsmesseinheit der Bewegungsgeschwindigkeit des Probanden. Der Geschwindigkeitssensor sensorisiert allerdings nicht die Geschwindigkeit der Aktivitätsmesseinheit selbst, sondern die Geschwindigkeit des Probanden, wenn die Aktivitätsmesseinheit nicht direkt am Probanden getragen werden, sondern dessen Bewegungsaktivität aus der Ferne erfassen soll.

Dann kann beispielsweise der Geschwindigkeitssensor aus einem Bewegungsmelder und einem Zeitsensor bestehen, sodass aus einem mithilfe des Bewegungsmelders ermittelten, vom Probanden zurückgelegten Weg und einer mithilfe des Zeitsensors gemessenen entsprechenden Zeit der Bewegung eine Bewegungsgeschwindigkeit des Probanden messbar bzw. bestimmbar ist. Hier wird auch ein Bestimmen oder Errechnen der Geschwindigkeit durch sekundäre Parameter wie einem Weg und einer Zeit als Messung verstanden, da die Aktivitätsmesseinheit dazu geeignet ist, die motorische Bewegungsaktivität zu erfassen und entsprechende Daten bereitzustellen und weiterzuliefern.

Die Aktivitätsmesseinheit kann beispielsweise auch eine GPS- oder Mobilfunk-basierte Positionserfassungseinheit aufweisen, sodass etwa lineare oder nichtlineare Bewegungen des Probanden mit Hilfe einer satellitengestützten Positionserfassung oder einer Mehrpunktberechnung anhand von Positionsdaten von Mobilfunkmasten und der Positionserfassungseinheit der Aktivitätsmesseinheit oder des Probanden messbar sind, um so die Bewegungsaktivität des Probanden zu bestimmen. Dabei soll - wie oben bereits beschrieben - die tatsächliche motorische Bewegungsaktivität des Probanden gemessen werden, sodass eine Positionserfassungseinheit vorzugsweise mit einem oder mehreren der zuvor genannten Sensoren der Aktivitätsmesseinheit gemeinsam betrieben werden, um passive Bewegungen des Probanden nicht als aktive motorische Bewegungen des Probanden zu interpretieren.

Einige der physikalischen Parameter, die der motorischen Bewegungsaktivität des Probanden zugrunde liegen, welche mit der Aktivitätsmesseinheit messbar sind, können beim Auftreten einer Exazerbation im Rahmen einer Lungenerkrankung des Probanden einer Veränderung unterliegen. Es ist jedoch auch möglich, dass sich dieselben oder andere Parameter bereits im Vorfeld eines Auftretens einer Exazerbation verändern. Darüber hinaus kann sich eine motorische Bewegungsaktivität des Probanden auch auf seine Lungenatmung auswirken, sowie die Lungenatmung sich auch auf seine motorische Bewegungsaktivität auswirken kann. Somit können die angegebenen Parameter der Lungenatmung sowie die motorische Bewegungsaktivität bei einer Exazerbation oder im Vorfeld einer Exazerbation veränderlich und sind für eine Prognose eines Auftretens einer Exazerbation relevant sein.

Gemäß den Beschreibungen der Spirometereinheit und der Aktivitätsmesseinheit sind Exazerbations-relevante Parameter also beispielsweise ein Aus- bzw. Einatemvolumen bei einem Atemvorgang, das gesamte Lungenvolumen des Probanden, ein Atemdruck, eine Flussgeschwindigkeit, ein Massefluss und ein Volumenfluss sowie lineare, nichtlineare und rotatorische Bewegungen bzw. Beschleunigungen, eine Kraft, eine Beschleunigung, eine Dauer, eine Richtung, ein Weg, eine Geschwindigkeit, ein Drehimpuls oder ein Drehmoment von aktiven Bewegungen des Probanden bzw. seiner Körperteile. Aus einzelnen dieser Parameter ist in der Regel keine Prognose über ein Eintreten einer Exazerbation möglich. Dennoch können diese Parameter für eine Prognose einer Exazerbation relevant sein. Insbesondere kann eine längerfristige Zusammenschau mehrerer dieser Parameter - obwohl sie für sich genommen nicht aussagekräftig sind - eine entsprechende Diagnose ermöglichen und somit eine entsprechende Prognose über den Zeitpunkt eines Auftretens einer Exazerbation erlauben. Dabei ist eine Zusammenschau von durch die Spirometereinheit gelieferten Parametern und von durch die Aktivitätsmesseinheit gelieferten Parametern besonders als Grundlage für eine Prognose geeignet.

Zum Sammeln der Messdaten der Spirometereinheit und der Aktivitätsmesseinheit weist die Vorrichtung eine Auswerteeinheit auf. Die Auswerteeinheit sammelt die Messdaten der Spirometereinheit und der Aktivitätsmesseinheit zentral und fasst sie zusammen, speichert sie also beispielsweise oder stellt sie für einen Abruf oder eine folgende Datenverarbeitung bereit.

Zu diesem Zweck sind die Spirometereinheit, die Aktivitätsmesseinheit und die Auswerteeinheit so miteinander verbunden, dass eine Übertragung der Messdaten zwischen diesen drei Einheiten möglich ist. Dazu können diese Einheiten beispielsweise über ein drahtgebundenes oder ein drahtloses Datenkommunikationsnetz miteinander verbunden sein. Ein drahtgebundenes Datenkommunikationsnetz kann beispielsweise durch eine Verkabelung der Spirometereinheit, der Aktivitätsmesseinheit und der Auswerteeinheit über Strom und somit Daten leitende Kabel wie beispielsweise Kupferkabel erreicht werden. Beispiele für drahtlose Datenkommunikationsnetze sind Infrarot- oder Funk-basierte Datennetze, welche eine Übertragung von Messdaten zwischen den Einheiten über Licht- bzw. Funkwellen, also über eine Ausbreitung elektromagnetischer Wellen ermöglichen.

Mit der Vorrichtung können beispielsweise die Spirometereinheit und die Aktivitätsmesseinheit zu einem ersten Zeitpunkt Messdaten an die Aktivitätsmesseinheit übertragen, sodass diese Messdaten zu diesem ersten Zeitpunkt in der Auswerteeinheit gesammelt werden. Für eine Betrachtung der Messdaten der Spirometereinheit und der Aktivitätsmesseinheit kann es vorteilhaft sein, zu den Messdaten zugehörige Zeitpunkte mit zu erfassen, abzuspeichern und/oder diesen zuzuordnen. Zu diesem Zweck kann die Vorrichtung einen Zeitgeber aufweisen. Dadurch ist es möglich, jedem Messwert der Spirometereinheit sowie der Aktivitätsmesseinheit einen entsprechenden Zeitpunkt zuzuordnen. So ist beispielsweise bei einer Vielzahl von Messwerten der Spirometereinheit und der Aktivitätsmesseinheit eine spätere Betrachtung der Messwerte über einen Zeitraum oder für bestimmte Zeitpunke möglich. Die Vorrichtung kann mit der Spirometereinheit beispielsweise die Lungenatmung des Probanden zu mehreren Zeitpunkten bzw. über mehrere Zeiträume hinweg messen und diese Messwerte in der Auswerteeinheit mitsamt den zugehörigen Zeitangaben speichern. Dadurch liegen für eine spätere Analyse ausreichend viele Messwerte für eine hohe Aussagekraft über die Lungenatmung des Probanden vor. Ebenso kann mit der Aktivitätsmesseinheit eine motorische Bewegungsaktivität des Probanden über einen längeren Zeitraum, beispielsweise über Wochen oder Monate erfasst werden und die entsprechenden Messwerte sind durch die Zuordnung von Zeitwerten im Nachhinein stets zeitlich rekonstruierbar.

Es ist auch möglich, dass eine Vielzahl von Messungen mit der Spirometereinheit oder der Aktivitätsmesseinheit über einen Tagesverlauf jeweils zu einem Wert für die Lungenatmung bzw. die motorische Bewegungsaktivität zusammengefasst und einem entsprechenden Zeitpunkt bzw. Zeitwert des Zeitgebers zugeordnet wird. Dies geschieht vorzugsweise durch die Auswerteeinheit. So kann beispielsweise bei einer hohen motorischen Bewegungsaktivität des Probanden über einen gesamten Tag hinweg ein einzelner, entsprechend hoher Wert für die motorische Bewegungsaktivität bestimmt werden und diesem Wert der jeweilige Tag etwa in Form des entsprechenden Datums als Zeitwert zugeordnet werden. An einem anderen Tag, an dem der Proband beispielsweise im Bett liegt und sich kaum bewegt, wird die motorische Bewegungsaktivität dann zu einem entsprechend niedrigen Wert zusammengefasst und diesem der entsprechende Tag bzw. das Datum als Zeitwert zugeordnet. Entsprechende Zusammenfassungen von Messwerten über unterschiedliche Zeitverläufe wie etwa einem halben Tag oder einer ganzen Woche können selbstverständlich ebenso für die Messwerte der Spirometereinheit durchgeführt werden.

Beispielsweise werden innerhalb von 24 Stunden die Messwerte der Lungenatmung aus der Spirometereinheit zu zwei Werten zusammengefasst und zwei entsprechenden Zeitwerten zugeordnet, während ein Wert für die motorische Bewegungsaktivität des Probanden innerhalb von 24 Stunden bestimmt wird und einem entsprechenden Zeitwert des Tages zugeordnet wird. Das Bestimmen eines Wertes aus einer Vielzahl von Messwerten der Spirometereinheit oder der Aktivitätsmesseinheit zu einem entsprechenden aussagekräftigen Wert des jeweils zugrunde liegenden Zeitverlaufs kann, wie oben beschrieben, durch die Auswerteeinheit erfolgen.

Beispielsweise bestimmt die Auswerteeinheit einen ersten Wert für die Lungenatmung des Probanden um 8.00 Uhr morgens, wobei der entsprechende Zeitwert für 8.00 Uhr morgens des Tages diesem Wert bzw. Tageswert zugeordnet und mit diesem zusammen abgespeichert wird. Dabei liegen diesem Wert Messungen der Lungenatmung des Probanden durch die Spirometereinheit der entsprechenden vorangegangenen Nacht zugrunde oder aber der exakte Messwert um 8.00 Uhr. Ferner wird beispielsweise um 18.00 Uhr abends desselben Tages ein zweiter Wert bzw. Tageswert für die Lungenatmung des Probanden - wiederum auf der Basis des vorangegangenen Zeitraums, hier etwa zwischen 8.00 Uhr und 18.00 Uhr, oder des exakten Messwerts um 18.00 Uhr - bestimmt und der entsprechende Zeitwert für 18.00 Uhr abends dieses Tages diesem zweiten Wert zugeordnet. Zum Beispiel wird ebenfalls zu diesem zweiten Zeitpunkt, also um 18.00 Uhr abends, durch die Auswerteeinheit ein Wert einer motorischen Bewegungsaktivität des entsprechenden Tages auf der Grundlage der Messwerte der Aktivitätsmesseinheit der vorangehenden 24 Stunden eingeholt dem Zeitwert für 18.00 Uhr abends dieses Tages zugeordnet. Auf diese Weise sammelt die Vorrichtung an jedem Tag zwei der Lungenatmung des Probanden entsprechende Werte und einen der motorischen Bewegungsaktivität des Probanden entsprechende Werte, sodass diese drei Werte untereinander und auch mit entsprechenden Werten vorangegangener Tage in einer Zusammenschau betrachtet und verglichen werden können.

Die Betrachtung von Veränderungen dieser Werte über Tage und Wochen hinweg kann dabei eine Diagnose eines Arztes bzw. eine Prognose einer Exazerbation im Rahmen einer Lungenerkrankung ermöglichen. Eine Exazerbation kann mit einem herkömmlichen Spirometer oder Pneumotachographen nicht vorausgesagt werden. Zwar ist eine Verschlechterung der Lungenatmung des Probanden durch die Betrachtung entsprechender punktueller oder zusammengefasster Messwerte mit entsprechenden vorangehenden Werten möglich und ein negativer Trend in der Bewegungsaktivität kann auf eine erhöhte Wahrscheinlichkeit einer nahenden Exazerbation hindeuten. Allerdings können die Werte für die Lungenatmung des Probanden auch kurzfristig durch eine Einnahme von Medikamenten oder durch eine überdurchschnittliche hohe körperliche Anstrengung des Probanden oder auch aufgrund von psychischer Belastung einen negativen Trend zeigen, obwohl in diesen Fällen keine grundlegende medizinische Indikation und somit also kein Anlass zur Besorgnis besteht, da eine Exazerbation tatsächlich nicht zu erwarten ist und sich der Gesundheitszustand des Probanden womöglich sogar tatsächlich verbessert. Eine Korrelation der Werte für die Lungenatmung mit der motorischen Bewegungsaktivität erlaubt jedoch eine weit verlässlichere Prognose bzw. Diagnose über eine zu erwartende Exazerbation als bisher bekannte Verfahren bzw. Vorrichtungen und kann insbesondere zusammen mit einer entsprechenden medizinischen Fachkenntnis und Erfahrung eines Arztes eine zuverlässige Prognose über den Zeitpunkt eines Auftretens einer Exazerbation erlauben.

Die Spirometereinheit, die Aktivitätsmesseinheit und die Auswerteeinheit können miteinander verbunden und wieder voneinander getrennt werden, sind also lösbar aneinander befestigbar. Somit ist die Vorrichtung zerlegbar, sodass beispielsweise bei dem Ausfall einer der drei Einheiten eine entsprechende Ersatzeinheit eingesetzt werden kann. Auch ist es so möglich, dass beispielsweise die Spirometereinheit von der Aktivitätsmesseinheit und der Auswerteeinheit abgetrennt wird, um Reinigungs- oder Wartungsmaßnahmen zu erleichtern.

Beispielsweise kann die Aktivitätsmesseinheit von der Spirometereinheit aufgenommen werden und in dieser lösbar befestigt werden. Dann weist die Spirometereinheit einen entsprechenden Raum für die Aktivitätsmesseinheit auf, sodass die gesamte Vorrichtung kompakt bleibt.

Für eine später beispielsweise durch einen Arzt zu vollziehende Diagnose anhand der Messdaten der Lungenatmung des Probanden und der Messdaten seiner motorischen Bewegungsaktivität sind diese Messdaten mit der Auswerteeinheit sammelbar und gegenüberstellbar, können also zusammen bereitgestellt werden. Dazu können Datensätze mit den Messdaten der Spirometereinheit bzw. der Aktivitätsmesseinheit zu einem übergeordneten Datensatz zusammengefasst werden, so dass die Messdaten gesammelt bereitstellbar sind.

Auch beim Vergleichen der ermittelten Messwerte der motorischen Bewegungsaktivität bzw. der Lungenatmung können zusammengefasste Datensätze durch die Auswerteeinheit erstellt werden, welche jeweils Ergebnisse aus Vergleichen darstellt. So können beispielsweise alle über einen gesamten Tag erfassten Messwerte der Spirometereinheit und der Aktivitätsmesseinheit sowie daraus bereits zu Datensätzen zusammengefassten Messwerte zu einem einzigen Datensatz je Zeitraum, also in diesem Beispiel je Tag, zusammengefasst werden. Ein Datensatz für einen Zeitraum kann jedoch beispielsweise auch nur in einem Maximalwert des entsprechenden Zeitraums, also beispielsweise eines Tages, bestehen. So entspricht ein ermittelter Wert für eine Lungenatmung des Probanden an einem bestimmten Tag beispielsweise dem Maximalwert des Ausatemdruckes des Tages.

Damit alle korrelierten, verglichenen, zusammengefassten bzw. ermittelten Messdaten, Werte oder Datensätze von der Auswerteeinheit ausgebbar sind, kann die Vorrichtung eine entsprechende Ausgabeeinheit aufweisen. Diese kann beispielsweise ein graphisches Anzeigeelement, eine LED-Anordnung, einen Bildschirm, oder andere optische Anzeigevorrichtungen oder etwa akustische oder sensorische Ausgabeeinheiten aufweisen, wie zum Beispiel Lautsprecher bzw. Vibrationsgeber. Damit außerdem die korrelierten, verglichenen, zusammengefassten oder ermittelten Messdaten, Werte oder Datensätze speicherbar sind, weist die Vorrichtung vorteilhafterweise eine Speichereinheit auf, wie etwa einen flüchtigen oder nichtflüchtigen Schreib- oder Schreiblesespeicher, z.B. ein DRAM, ein EEPROM oder eine Festplatte. Die Speichereinheit ist vorteilhafter Weise in der Auswerteeinheit angeordnet. Sämtliche dieser Daten, insbesondere jedoch zusammengefasste Werte oder Datensätze, können außerdem von der Vorrichtung an eine oder mehrere weitere, externe Einheit weitergeleitet werden.

Die Vorrichtung in allen ihren beschriebenen Weiterentwicklungen ist vorteilhafter Weise tragbar, sodass sie auf einfache Weise von einem Probanden mitgeführt werden kann. Beispielsweise weist die Vorrichtung die Größe eines handelsüblichen Spirometergeräts auf, sodass im Vergleich zu einer Benutzung eines Spirometers die Verwendung der erfindungsgemäßen Vorrichtung keine gewichts- oder größenbedingte Nachteile für einen Benutzer bzw. den Probanden auftreten.

Zu diesem Zweck weist die Vorrichtung vorteilhafterweise eine Energiequelle auf, welche zum Bereitstellen aller für den Betrieb der entsprechenden Sensorik oder Elektronik notwendigen Energie ausreicht. Unter einer Energiequelle seien hier sowohl Energiespeicher als auch Energiewandler verstanden, also beispielsweise sowohl Batterien als auch Solarzellen.

Damit die Spirometereinheit, die Auswerteeinheit und die Aktivitätsmesseinheit voneinander lösbar sind und zugleich autonom betrieben werden können, weist vorteilhafterweise jede dieser Einheiten eine eigene Energiequelle auf. So kann beispielsweise die Spirometereinheit von den anderen beiden Einheiten gelöst und betrieben werden. Unabhängig davon kann dann die Aktivitätsmesseinheit zum Messen der motorischen Bewegungsaktivität des Probanden genutzt werden, ohne mit der Spirometereinheit oder der Auswerteeinheit verbunden bzw. an diesen Einheiten befestigt zu sein. Um Messdaten gesammelt an die Auswerteeinheit übertragen zu können, kann sowohl die Aktivitätsmesseinheit als auch die Spirometereinheit eine eigene Speichereinheit aufweisen.

Die Vorrichtung kann ferner eine optische Anzeige aufweisen, beispielsweise in Form einer LED-Leiste mit LEDs in verschiedenen Farben. Vorzugsweise weist die Aktivitätsmesseinheit diese Anzeige auf, wobei die LEDs dem Probanden anzeigen können, wie hoch oder intensiv seine motorische Bewegungsaktivität ist. Beispielsweise können rote LEDs darauf hindeuten, dass die motorische Bewegungsaktivität des Probanden sehr gering ist, was dieser beispielsweise als Aufforderung verstehen soll, sich mehr zu bewegen. Eine entsprechende Anzeige grüner LEDs kann dem Probanden dementsprechend signalisieren, dass seine körperliche Aktivität in einem wünschenswerten Umfang erfolgt. Im Wesentlichen dient eine derartige Anzeige einer Rückmeldung über durch die Aktivitätseinheit aufgenommene Messdaten an den Probanden oder an eine weitere Person.

In einem erfindungsgemäßen Verfahren zum Erfassen von Exazerbations-relevanten Parametern eines Probanden wird eine Lungenfunktion des Probanden mit einer Spirometereinheit zu einem ersten Zeitpunkt an einem ersten Tag gemessen. Zu einem zweiten Zeitpunkt des ersten Tages wird eine zweite Messung einer Lungenfunktion des Probanden durchgeführt. Die Spirometereinheit misst dabei - wie oben beschrieben - die Lungenfunktion des Probanden und demnach Parameter wie ein Lungenvolumen, Atemvolumina, Atemdrücke, Masse- und/oder Volumenflüsse. Zudem wird zu einem dritten Zeitpunkt des ersten Tages mit einer Aktivitätseinheit eine motorische Bewegungsaktivität des Probanden in einem ersten Zeitraum gemessen. Der erste Zeitraum entspricht dabei vorzugsweise der Dauer eines Tages. Wie oben beschrieben, kann die Aktivitätsmesseinheit dazu Parameter wie eine Stärke, eine Richtung oder eine Häufigkeit von Bewegungen des Probanden bzw. seiner Extremitäten und seines Rumpfes messen. Außerdem werden die dem ersten und zweiten Zeitpunkt des ersten Tages entsprechenden Messdaten der Spirometereinheit und die dem dritten Zeitpunkt des ersten Tages der Aktivitätsmesseinheit in einer Auswerteeinheit gespeichert. Somit liegen in der Auswerteeinheit drei Messwerte für den ersten Tag vor.

Dazu kann eine Information über eine Veränderung zwischen den Messwerten der ersten und zweiten Messung der Lungenfunktion mit der Spirometereinheit in der Auswerteeinheit bestimmt werden und für einen Benutzer oder weitere Einheiten oder Vorrichtungen als Datensatz bereitgestellt werden.

Zudem können ein drittes Messen einer Lungenfunktion des Probanden mit der Spirometereinheit zu einem ersten Zeitpunkt eines zweiten Tages sowie ein viertes Messen einer Lungenfunktion des Probanden mit der Spirometereinheit zu einem zweiten Zeitpunkt des zweiten Tages durchgeführt werden. Daneben kann ein zweites Messen einer motorischen Bewegungsaktivität des Probanden über einen der Dauer des ersten Zeitraums entsprechenden zweiten Zeitraum mit der Aktivitätsmesseinheit zu einem dritten Zeitpunkt des zweiten Tages durchgeführt werden, wonach die Messdaten der Spirometereinheit und der Aktivitätsmesseinheit dann ebenfalls in der Auswerteeinheit gespeichert werden. Somit liegen jeweils drei Messwerte für jeden der beiden Tage in der Auswerteeinheit vor.

Folglich können sowohl eine Information über eine Veränderung zwischen der ersten und dritten Messung der Lungenfunktion mit der Spirometereinheit in der Auswerteeinheit als auch eine Information über eine Veränderung zwischen der zweiten und vierten Messung mit der Spirometereinheit in der Auswerteeinheit bestimmt und bereitgestellt werden. Außerdem kann eine Information über eine Veränderung zwischen der ersten und zweiten Messung mit der Aktivitätsmesseinheit in der Auswerteeinheit bestimmt und bereitgestellt werden.

Aufgrund der in der Auswerteeinheit vorliegenden Datensätze kann auch eine Information über eine Veränderung zwischen den Messwerten der dritten und vierten Messung der Lungenfunktion mit der Spirometereinheit in der Auswerteeinheit erstellt und abgespeichert werden.

In den oben dargestellten Messungen an einem ersten bzw. einem zweiten Tag kann beispielsweise der erste Zeitpunkt des ersten Tages der Uhrzeit 8.00 Uhr des ersten Tages und der zweite Zeitpunkt des ersten Tages der Uhrzeit 18.00 Uhr des ersten Tages entsprechen. Der dritte Zeitpunkt des ersten Tages kann mit dem zweiten Zeitpunkt des ersten Tages zusammenfallen und beispielsweise ebenfalls der Uhrzeit 18.00 Uhr des ersten Tages entsprechen. Weitere, dem ersten, zweiten und dritten Zeitpunkt des ersten Tages entsprechende erste, zweite bzw. dritte Zeitpunkte weiterer Tage entsprechen vorzugsweise den gleichen Uhrzeiten der Zeitpunkte des ersten Tages, also beispielsweise wiederum 8.00 Uhr, 18.00 Uhr und ebenfalls 18.00 Uhr. Dadurch weisen Zeiträume zwischen den entsprechenden Zeitpunkten aufeinander folgender Tage jeweils die Dauer eines Tages auf.

Diese Messungen können entsprechend über zahlreiche Tage, Wochen und Monate durchgeführt werden, sodass die Auswerteeinheit einen Datenbestand mit einer großen Menge an Messdaten sammeln kann. Dadurch sind entsprechend die Lungenfunktion und die motorische Bewegungsaktivität über große Zeiträume beobachtbar und Veränderungen darin wesentlich besser erfassbar als bei lediglich seltenen punktuellen Messungen.

Es kann vorkommen, dass Zeitpunkte bzw. die Tageszeiten der Messungen unterschiedlicher Tage voneinander abweichen, obwohl ein Benutzer dazu angehalten ist, die vorgegebenen Zeitpunkte einzuhalten. Beispielsweise kann die zweite Messung mit der Spirometereinheit am ersten Tag um 18.00 Uhr, die vierte Messung mit der Spirometereinheit am zweiten Tag aber erst um 20.00 Uhr durchgeführt werden. Dann könnte eine Information über eine Veränderung zwischen der zweiten und vierten Lungenfunktionsmessung weniger aussagekräftig für eine Entwicklung eines Lungenzustandes sein, als wenn diese beiden Messungen zur gleichen Tages- bzw. Uhrzeit am ersten bzw. zweiten Tag durchgeführt würden. In solch einem Fall kann ein Rückschluss auf einen für 18.00 Uhr eines Tages zu erwartenden Messwert aus einem vorangehenden Messwert desselben Tages gezogen werden. Dieser Rückschluss erlaubt insofern eine Korrektur des abzuspeichernden Messwerts, als ein Schätzwert für 18.00 Uhr anstelle eines tatsächlich gemessen Wertes abgespeichert wird. Dazu kann die Auswerteeinheit beispielsweise eine Linearisierung über den Wert für 8.00 Uhr und dem Wert für 20.00 Uhr vornehmen, um daraus einen Schätzwert für 18.00 Uhr des aktuellen Tages zu errechnen. Dieser Schätzwert wird dann in der Auswerteeinheit als für 18.00 Uhr des besagten Tages gemessener Wert abgespeichert und bereitgestellt.

Diese und weitere Vorteile und Merkmale der Erfindung werden nachfolgend anhand von Beispielen unter der Zuhilfenahme der begleitenden Figuren näher erläutert. Es zeigen:
- **Fig. 1**: eine schematische Skizze einer erfindungsgemäßen Vorrichtung in einer Ausführungsform der Erfindung und
- **Fig. 2**: beispielhafte Messdiagramme von durch die erfindungsgemäße Vorrichtung erfassten Messdaten.

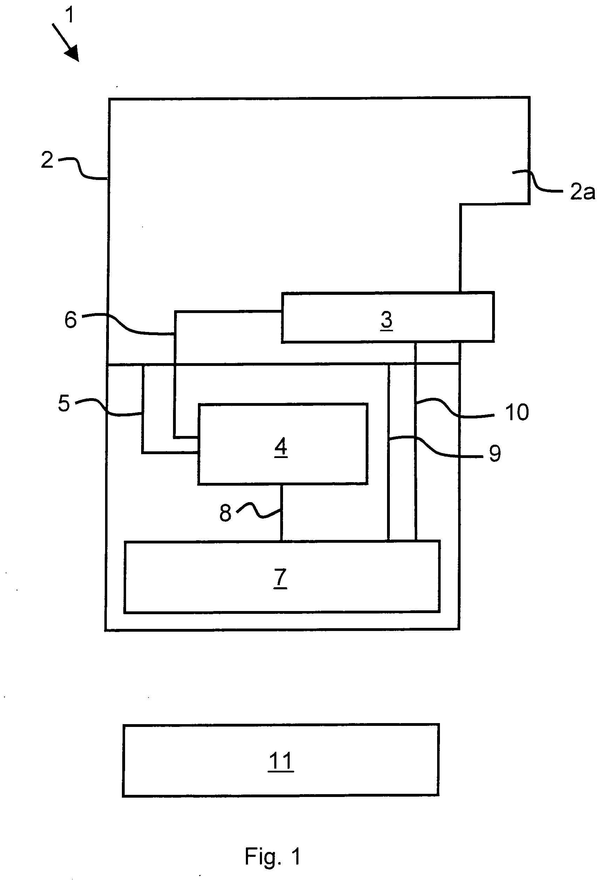

Fig. 1 zeigt eine Vorrichtung 1 in einer Ausführungsform der Erfindung. Die Vorrichtung 1 weist eine Spirometereinheit 2 zum Messen einer Lungenatmung eines Probanden auf. Zu diesem Zweck kann ein Proband durch ein Mundstück 2a in die Spirometereinheit 2 hinein ausatmen oder aus ihr hinaus einatmen. Die Spirometereinheit 2 kann dabei das Lungenvolumen sowie die Ein- und Ausatemvolumina des Probanden messen. Die Spirometereinheit 2 kann aber auch einen Volumenfluss, einen Massefluss, eine Luftfeuchtigkeit und eine Temperatur messen, wobei auch Maximal- und Minimalwerte sowie über einen einstellbaren Zeitverlauf gemittelte Werte des Volumenflusses bzw. des Masseflusses messbar sind. Zu diesem Zweck weist die Spirometereinheit 2 einen Volumensensor, einen Volumenflusssensor, einen Masseflusssensor, einen Drucksensor, einen Temperatursensor und einen Feuchtigkeitssensor auf (in der Fig. nicht gezeigt).

Die Vorrichtung 1 weist zudem eine Aktivitätsmesseinheit 3 auf, welche bei der dargestellten Ausführungsform von der Spirometereinheit 2 aufgenommen und in dieser lösbar befestigt ist. Des Weiteren weist die Vorrichtung 1 eine Auswerteeinheit 4 auf, welche über eine erste Datenverbindung 5 mit der Spirometereinheit 2 und über eine zweite Datenverbindung 6 mit der Aktivitätsmesseinheit 3 verbunden ist. Die erste und die zweite Datenverbindung 5, 6 stehen dabei stellvertretend sowohl für drahtgebundene Datenstrecken wie beispielsweise elektrisch leitende Kabel, als auch für drahtlose Datenstrecken wie etwa Infrarot- oder Funk-Datenübertragungsstrecken. In jedem Fall ist über die erste und zweite Datenverbindung 5, 6 eine Datenübertragung zwischen der Spirometereinheit 2, der Aktivitätsmesseinheit 3 und der Auswerteeinheit 4 möglich, so dass Messdaten der Spirometereinheit 2 und der Aktivitätsmesseinheit 3 beispielsweise an die Auswerteeinheit 4 geliefert werden können.

Da die Aktivitätsmesseinheit 3 von der Spirometereinheit 2 aufnehmbar und in dieser befestigt ist, ist es auch möglich, dass in einer weiteren Ausführungsform der Erfindung über eine erste Datenverbindung 5 zwischen der Spirometereinheit 2 und der Auswerteeinheit 4 auch Daten zwischen der Aktivitätsmesseinheit 3 und der Auswerteeinheit 4 übertragbar sind. Dann besteht ein direkter Datenaustausch zwischen der Spirometereinheit 2 und der Aktivitätsmesseinheit 3.

Die Aktivitätsmesseinheit 3 kann eine motorische Bewegungsaktivität eines Probanden messen. Bei der gezeigten Ausführungsform der Vorrichtung 1 sind unterschiedliche Anwendungsmöglichkeiten der Aktivitätsmesseinheit 3 gegeben. Nicht alle dieser Möglichkeiten müssen in allen möglichen weiteren Ausführungsformen der Vorrichtung 1 gegeben sein, jedoch sind die hier aufgezeigten Anwendungsmöglichkeiten beispielhaft. Jedenfalls ist mit der Aktivitätsmesseinheit 3 stets eine Messung der aktiven motorischen Bewegungsaktivität eines Probanden möglich. Mit der Aktivitätsmesseinheit 3 sind beispielsweise eine Kraft, eine Beschleunigung, eine Dauer, eine Richtung, ein Weg, eine Geschwindigkeit, ein Drehimpuls und/oder ein Drehmoment einer Bewegung der Aktivitätsmesseinheit oder des Probanden messbar. Zu diesem Zweck weist die Aktivitätsmesseinheit 3 eine Vielzahl von Sensortypen oder Sensorsystemen auf, wie beispielsweise einen Luftdrucksensor, ein Gyroskop, einen Bewegungssensor, einen Beschleunigungssensor, einen Geschwindigkeitssensor, einen Laserkreisel oder einen Faserkreisel oder eine GPS- oder Mobilfunk-basierte Positionserfassungseinheit.

Ist die Aktivitätsmesseinheit 3 von der Spirometereinheit 2 der Vorrichtung 1 aufgenommen und in dieser befestigt, so kann die Aktivitätsmesseinheit 3 alle Bewegungen der gesamten Vorrichtung 1 nachvollziehen und dadurch messen, welchen Bewegungen, also beispielsweise welchen Linearbewegungen, Rotationen oder Beschleunigungen die Vorrichtung 1 bzw. die Aktivitätsmesseinheit 3 ausgesetzt ist. Ist bekannt, dass die Vorrichtung 1 bzw. die Aktivitätsmesseinheit 3 am Körper des Probanden getragen wird, ist somit ein direkter Rückschluss auf die motorische Bewegungsaktivität des Probanden möglich.

Die Auswerteeinheit 4 sammelt die Messdaten aus der Spirometereinheit 2 und der Aktivitätsmesseinheit 3 und kann diese speichern. Daneben können die Messdaten beispielsweise mit Methoden der Datenverarbeitung verarbeitet werden, also etwa zu übergeordneten Datensätzen zusammengefasst, für eine grafische Darstellung der Messdaten interpretiert und aufbereitet oder zum elektronischen Versenden der Daten in entsprechende Dateien mit festgelegten Dateiformaten konvertiert werden.

Die Vorrichtung 1 weist zudem eine Energiequelle 7 auf. In der gezeigten Ausführungsform ist die Energiequelle 7 eine Batterie, sie kann jedoch auch in weiteren Formen von Energiespeichern oder Energiewandlern ausgeführt sein, etwa als Kondensator oder Solarzelle. Die Energiequelle 7 versorgt die Auswerteeinheit 4 über einen ersten Energieleiter 8, die Spirometereinheit 2 über einen zweiten Energieleiter 9 und die Aktivitätsmesseinheit 3 über einen dritten Energieleiter 10 mit Energie, so dass diese drei Einheiten 2, 3, 4 mit einer jeweils ausreichenden Energie zu deren Betrieb versorgt werden können. Die drei Energieleiter 8, 9, 10 stehen dabei stellvertretend für drahtgebundene oder drahtlose Energieübertragungsstrecken und können beispielsweise als Strom leitende Kupferkabel bzw. als elektromagnetische Felder für eine auf einer elektromagnetischen Induktion basierende Energieübertragung ausgeführt sein. Es ist jedoch auch möglich, dass die Spirometereinheit 2, die Aktivitätsmesseinheit 3 sowie die Auswerteeinheit 4 zusätzlich zur Energiequelle 7 oder an ihrer Stelle jeweils eine eigene Energiequelle (nicht gezeigt) aufweisen.

Die Vorrichtung 1 ist durch die Energiequelle 7 unabhängig von einem lokalen Energienetz betreibbar. Zudem ist die Vorrichtung 1 ein tragbares Handgerät und entsprechend klein und leicht, so dass sie von einem Probanden auf einfache Weise transportiert und benutzt werden kann.

Außerhalb der Vorrichtung 1 ist eine externe Empfangseinheit 11 angeordnet. An diese kann die Vorrichtung 1 die gesammelten Messdaten beispielsweise über eine drahtlose Datenkommunikationsverbindung übertragen.

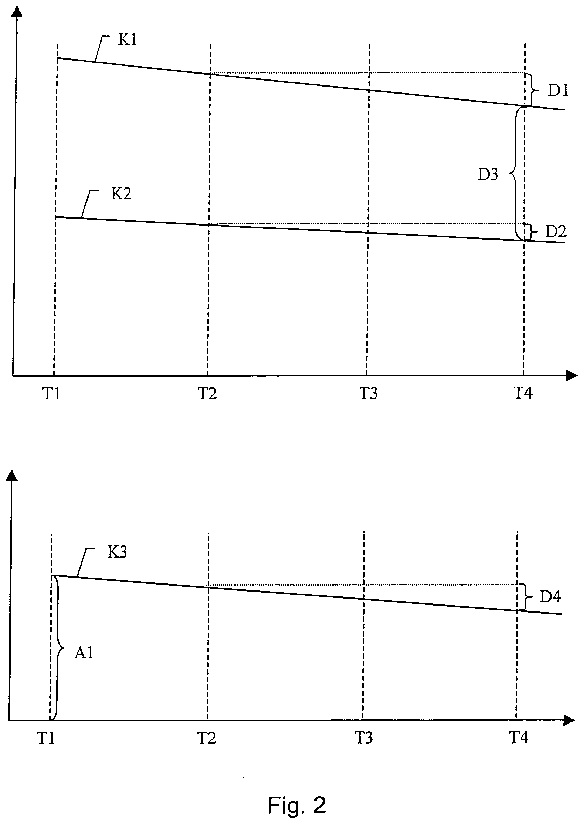

Die Fig. 2 zeigt beispielhafte Messdiagramme von durch eine erfindungsgemäße Vorrichtung erfassten Messdaten. Anhand dieser Messdiagramme werden die Wirkungsweise der Vorrichtung sowie das entsprechende Verfahren zum Erfassen von Exazerbations-relevanten Parametern im Folgenden näher erläutert.

Im oberen Diagramm von Fig. 2 sind über der Abszissenachse Zeitpunkte bzw. Zeitverläufe angetragen, während die Ordinatenachse so genannte PEF (Peak Expiratory Flow)-Werte angibt. PEF-Werte entsprechen dem maximalen Volumenfluss eines Ausatemvorgangs eines Probanden und werden mithilfe der Spirometereinheit der Vorrichtung gemessen. Beispielsweise sind in dem oberen Diagramm von Fig. 2 zwei Kurven Kl, K2 dargestellt, wobei die erste Kurve K1 der Tageszeit bzw. Uhrzeit 8.00 Uhr und die zweite Kurve K2 der Uhrzeit 18.00 Uhr zugeordnet sind. Das bedeutet, das beispielsweise die Kurve K1 durch mit der Spirometereinheit gemessene PEF-Werte um jeweils 8.00 Uhr der Tage T1, T2, T3 bzw. T4 bestimmt ist. Ebenso ist die Kurve K2 durch mit der Spirometereinheit um jeweils 18.00 Uhr der Tage T1, T2, T3 bzw. T4 gemessene PEF-Werte bestimmt. Liegen PEF-Werte von mindestens zwei Tagen vor, sind Veränderungen oder Unterschiede der PEF-Werte von verschiedenen Tagen ermittelbar. Beispielsweise stellt der Differenzwert D1 den Unterschied der PEF-Werte für 8.00 Uhr des zweiten Tages T2 zum PEF-Wert für 8.00 Uhr des vierten Tages T4 dar. Ebenso stellt der Differenzwert D2 den Unterschied der PEF-Werte für 18.00 Uhr des zweiten Tages T2 zum PEF-Wert für 18.00 Uhr des vierten Tages T4 dar. Der Differenzwert D3 entspricht der Differenz der PEF-Werte für 8.00 Uhr und 18.00 Uhr des vierten Tages T4.

Die einzelnen Messwerte der entsprechenden Tage T1, T2, T3, T4 werden in der Auswerteeinheit gesammelt und bereitgestellt, und die Kurven Kl, K2 sowie die Differenzwerte Dl, D2, D3 sind mit der Auswerteeinheit bestimmbar.

Auf ähnliche Weise sind im unteren Diagramm von Fig. 2 sind über der Abszissenachse wiederum Zeitpunkte bzw. Zeitverläufe angetragen, während die Ordinatenachse die motorische Bewegungsaktivität angibt. Die motorische Bewegungsaktivität des Probanden wird mit der Aktivitätsmesseinheit gemessen. Ein hoher Wert entspricht dabei einer hohen motorischen Bewegungsaktivität des Probanden, während ein niedriger Wert einer entsprechend geringeren motorischen Bewegungsaktivität entspricht. Die Aktivitätswerte für die vier Tage T1, T2, T3, T4, sind hier jeweils der Tageszeit 18.00 Uhr des entsprechenden Tages zugeordnet, sodass die aus diesen Werten bestimmte Kurve K3 ebenfalls der Tageszeit 18.00 Uhr entspricht. Der Aktivitätswert A1 der motorischen Bewegungsaktivität am ersten Tag T1 gibt dabei die zwischen 18.00 Uhr des dem ersten Tag 1 vorausgehenden Tages und 18.00 Uhr des ersten Tages T1 von dem Probanden geleistete motorische Bewegungsaktivität an.

Die der dritten Kurve K3 zugrunde liegenden Aktivitätswerte werden mit der Aktivitätsmesseinheit gemessen bzw. bestimmt und an die Auswerteeinheit weitergegeben, die diese Werte sammelt und bereitstellt. Sind mindestens zwei Aktivitätswerte in der Auswerteeinheit abgelegt, kann ein Differenzwert der Aktivitätswerte mit der Auswerteeinheit bestimmt werden, um eine Information über eine Veränderung der Werte über einen Zeitverlauf zu bieten. Beispielsweise gibt der Differenzwert D4 die Differenz der Aktivitätswerte des zweiten Tags T2 und des vierten Tags T4 an.

Die einzelnen Messwerte der Lungenfunktion bzw. die PEF-Werte sowie die Aktivitätswerte können für eine bestehende oder zukünftig eintretende Exazerbation relevante Parameter darstellen. Beispielsweise ist es möglich, dass ein eine Diagnose erstellender Arzt Veränderungen der Messwerte unterschiedlicher Tage durch die Bereitstellung der entsprechenden Daten durch die Auswerteeinheit erkennen kann und anhand eines längerfristigen Trends eine Prognose über ein Eintreten einer Exazerbation erstellen kann. In diesem Beispiel der Fig. 2 sind als Lungenfunktionswerte die PEF-Werte angegeben. Es können jedoch auch alle anderen, oben aufgezeigten Parameter der Spirometereinheit als Lungenfunktionswerte erfasst und entsprechend der hier genannten Zeitbetrachtungen und Berechnungen in der Auswerteeinheit verarbeitet werden. Eine alleinige Betrachtung der Entwicklung der Messwerte der Spirometereinheit bzw. der Aktivitätsmesseinheit ist üblicherweise nicht für eine genaue Prognose über ein zukünftiges Eintreten einer Exazerbation geeignet. Jedoch ist eine Zusammenschau der Lungenfunktionswerte mit den Aktivitätswerten weitaus besser für eine entsprechende Prognose bzw. einer zur Prognose führenden Diagnose geeignet.

Aus der Möglichkeit des Bestimmens einer Messkurve über Messwerte mehrerer Tage ergibt sich ein weiterer Vorteil des erfindungsgemäßen Verfahrens. Falls es dem Benutzer der Vorrichtung nicht möglich ist, an einem Tag eine Messung mit der Spirometereinheit um 18.00 h durchzuführen, sondern verspätet er sich beispielsweise mit einer Messung, so dass er diese erst um 20.00 Uhr vornimmt, ist durch die Auswerteeinheit eine Korrektur des entsprechenden PEF-Werts für 18.00 Uhr des aktuellen Tages möglich. Diese Korrektur besteht darin, mithilfe des PEF-Wertes für bzw. von 8.00 Uhr einen für 18.00 Uhr desselben, aktuellen Tages zu erwartenden PEF-Schätzwert zu errechnen. Dazu kann die Auswerteeinheit beispielsweise eine Linearisierung über den Wert für 8.00 Uhr und dem Wert für 20.00 Uhr vornehmen, um daraus einen PEF-Schätzwert für 18.00 Uhr des aktuellen Tages zu errechnen. Dieser PEF-Schätzwert wird dann in der Auswerteeinheit als PEF-Wert für 18.00 Uhr des besagten Tages abgespeichert und bereitgestellt.

Dadurch ist es einem Benutzer der Vorrichtung möglich, von empfohlenen Tageszeiten für die Messungen abzuweichen, wenn ihm eine entsprechende Einhaltung nicht möglich ist.

## Patentansprüche

1. Vorrichtung (1) zum Erfassen von Exazerbations-relevanten Parametern im Rahmen einer Lungenerkrankung eines Probanden, umfassend
- eine Spirometereinheit (2, 2a) zum Messen einer Lungenfunktion des Probanden;
- eine Aktivitätsmesseinheit (3) zum Messen einer Bewegungsstärke der Arme oder Beine relativ zum Rumpf des Probanden durch ein Messen der Beschleunigung oder Geschwindigkeit der Aktivitätsmesseinheit (3), wobei die Aktivitätsmesseinheit (3) dafür ausgelegt ist, direkt am Probanden getragen zu werden,
- eine Auswerteeinheit (4) zum Sammeln von Messdaten der Spirometereinheit (2, 2a) und der Aktivitätsmesseinheit (3), wobei die Auswerteeinheit (4) eingerichtet ist, Messdaten zusammenzufassen und diesen einen Zeitwert zuzuordnen, wobei eine Mehrzahl von durch die Aktivitätsmesseinheit (3) über einen Zeitraum von einem ganzen Tag gemessenen Messdaten zu einem Tageswert für die motorische Bewegungsaktivität mithilfe der Auswerteeinheit (4) zusammengefasst werden, und wobei die Auswerteeinheit (4) eingerichtet ist, an einem Tag von der Spirometereinheit (2, 2a) gemessene Messwerte der Lungenatmung zu zwei Lungenfunktionswerten zusammenzufassen und den zwei Lungenfunktionswerten zwei entsprechende Zeitwerte zuzuordnen; und
- einen Zeitgeber zum Erfassen, Zuordnen und/oder Abspeichern von zugehörigen Zeitwerten zu den Messdaten der Spirometereinheit (2, 2a) und der Aktivitätsmesseinheit (3),
wobei die zwei entsprechenden Zeitwerte der Lungenfunktionswerte zwei empfohlene Tageszeiten sind und die Auswerteeinheit (4) dazu eingerichtet ist, entsprechende geschätzte Lungenfunktionswerte für die empfohlenen Tageszeiten aus zu abweichenden Tageszeiten ermittelten Lungenfunktionswerten des aktuellen Tages durch eine Linearisierung zu errechnen.

2. Vorrichtung (1) gemäß Anspruch 1, wobei die Auswerteeinheit (4) dazu ausgebildet ist, einen Differenzwert (D3) der Lungenfunktionswerte für die zwei empfohlenen Tageszeiten zu bilden und diesem Differenzwert (D3) als Zeitwert einen entsprechenden Tag zuzuordnen.

3. Vorrichtung (1) gemäß einem der vorangehenden Ansprüche, wobei mit der Spirometereinheit (2, 2a) ein Atem- oder Lungenvolumen, eine Flussgeschwindigkeit, ein Massefluss und/oder ein Volumenflusses der Lungenatmung des Probanden messbar ist.

4. Vorrichtung (1) gemäß einem der vorangehenden Ansprüche, wobei die Spirometereinheit (2, 2a) einen Volumensensor, einen Volumenflusssensor, einen Masseflusssensor, einen Drucksensor, einen Temperatursensor und/oder einen Feuchtigkeitssensor aufweist.

5. Vorrichtung (1) gemäß einem der vorangehenden Ansprüche, wobei mit der Aktivitätsmesseinheit (3) eine Kraft, eine Beschleunigung, eine Dauer, eine Richtung, ein Weg, eine Geschwindigkeit, ein Drehimpuls und/oder ein Drehmoment einer Bewegung des Probanden messbar ist.

6. Vorrichtung (1) gemäß einem der vorangehenden Ansprüche, wobei die Aktivitätsmesseinheit (3) einen Massenträgheitssensor, ein Gyroskop, einen Bewegungssensor, einen Beschleunigungssensor, einen Geschwindigkeitssensor, einen Laserkreisel, einen Faserkreisel oder eine GPS- oder Mobilfunk-basierte Positionserfassungseinheit aufweist.

7. Vorrichtung (1) gemäß einem der vorangehenden Ansprüche, wobei die Spirometereinheit (2, 2a), die Aktivitätsmesseinheit (3) und die Auswerteeinheit (4) über ein drahtgebundenes oder ein drahtloses Kommunikationsnetz verbindbar sind, sodass eine Übertragung der Messdaten zwischen diesen Einheiten möglich ist.

8. Vorrichtung (1) gemäß einem der vorangehenden Ansprüche, wobei die Spirometereinheit (2, 2a), die Aktivitätsmesseinheit (3) und die Auswerteeinheit (4) lösbar aneinander befestigbar sind.

9. Vorrichtung (1) gemäß einem der vorangehenden Ansprüche, wobei mit der Auswerteeinheit (4) die Messdaten der Lungenatmung des Probanden und die Messdaten seiner motorischen Bewegungsaktivität für eine Diagnose gegenüberstellbar sind.

10. Vorrichtung (1) gemäß Anspruch 9, wobei die Gegenüberstellung über mit dem Zeitgeber erfassten, zugeordneten und/oder abgespeicherten Zeitwerte erfolgen kann.

11. Vorrichtung (1) gemäß einem der vorangehenden Ansprüche, wobei korrelierte, verglichene oder zusammengefasste Messdaten oder Datensätze von der Auswerteeinheit (4) über eine Ausgabeeinheit der Auswerteeinheit (4) ausgebbar, in einer Speichereinheit speicherbar oder an eine externe Einheit weiterleitbar sind.

12. Vorrichtung (1) gemäß einem der vorangehenden Ansprüche, wobei die Vorrichtung (1) tragbar ist.

13. Verfahren zum Erfassen von Exazerbations-relevanten Parametern mit einer Vorrichtung (1) nach einem der vorstehenden Ansprüche, wobei das Verfahren die folgenden Schritte umfasst:
- Messen eines Lungenfunktionswertes des Probanden mit der Spirometereinheit (2, 2a) zu einem ersten Zeitpunkt eines ersten Tages;
- Messen eines Lungenfunktionswertes des Probanden mit der Spirometereinheit (2, 2a) zu einem zweiten Zeitpunkt des ersten Tages;
- Messen einer motorischen Bewegungsaktivität der Arme oder Beine des Probanden über einen ersten Zeitraum mit der Aktivitätsmesseinheit (3) bis zu einem dritten Zeitpunkt des ersten Tages und Zusammenfassen der über einen Zeitraum von einem Tag gemessenen motorischen Bewegungsaktivität zu einem ersten zusammengefassten Wert;
- Speichern der Messdaten der Spirometereinheit (2, 2a) und des ersten zusammengefassten Werts in einer Auswerteeinheit (4);
- Errechnen entsprechender geschätzter Lungenfunktionswerte für empfohlene Tageszeiten aus zu abweichenden Tageszeiten gemessenen Lungenfunktionswerten des aktuellen Tages durch eine Linearisierung.

14. Verfahren nach Anspruch 13, wobei das Verfahren die zusätzlichen Schritte umfasst:
- Bilden eines Differenzwerts (D3) der Lungenfunktionswerte für die zwei empfohlenen Tageszeiten, und
- Zuordnen eines entsprechenden Tages diesem Differenzwert (D3) als Zeitwert.

## Claims

1. Device (1) for determining exacerbation-related parameters in the context of a pulmonary disease of a subject, comprising
- a spirometer unit (2, 2a) for measuring a lung function of the subject;
- an activity measurement unit (3) for measurement of a strength of movement of the arms or legs relative to the trunk of the subject by measurement of the acceleration or speed of the activity measurement unit (3), wherein the activity measuement unit (3) is designed to be carried directly on the subject,
- an evaluation unit (4) to collect measurement data of the spirometer unit (2, 2a) and the activity measurement unit (3), wherein die evaluation unit (4) is configured to summarise measured data and to assign a time value to this, wherein a plurality of measured data measured over a period of a whole day are summarised as a day value for the motor movement activity by the activity measurement unit (3) with the aid of the evaluiation unit (4), and wherein die evaluation unit (4) is arranged to summarise measured data of pulmonary respiration measured by the spirometer unit (2, 2a) on one day as two lung function values and to assign two corresponding time values to the two lung function values; and
- a timer for recording, allocating and/or storing of associated times to the measured data of the spirometer unit (2, 2a) and the activity measurement unit (3),
wherein the two corresponding times of the pulmonary function values are two recommended times of day and the evaluation unit (4) is arranged to calculate corresponding estimated pulmonary function values for the recommended times of day from the pulmonary function values determined at differing times of the current day by linearlization.

2. Device in accordance with claim (1), wherein die evaluation unit (4) is configured to form a difference value (D3) of pulmonary function values for the two recommended times of day and to assign this diffderence value (D3) to a corresponding day as time value.

3. Device (1) in accordance with one of the preceding claims, wherein a breath or lung volume, a flow rate, a mass flow and/or a volumetric flow of the pulmonary respiration of the subject can be measured with the spirometer unit (2, 2a).

4. Device (1) in accordance with one of the preceding claims, wherein die spirometer unit (2, 2a) comprises a volume sensor, a volumetric flow sensor, a mass flow sensor, a pressure sensor, a temperature sensor and/or a humidity sensor.

5. Device (1) in accordance with one of the preceding claims, wherein a force, an acceleration, a duration, a direction, a route, a speed, an angular momentum and/or a torque of a movement of the subject can be measured with the activity measurement unit (3).

6. Device (1) in accordance with one of the preceding claims, wherein the activity measurement unit (3) comprises a mass inertia sensor, a gyroscope, a movement sensor, an acceleration sensor, a speed sensor, a laser gyroscope, or a GPS or mobile radio-based position detection unit.

7. Device (1) in accordance with one of the preceding claims, wherein die spirometer unit (2, 2a), the activity measurement unit (3) and the evaluation unit (4) can be connected by means of a wired or a wireless communication network, so that it is possible to transmit measurement data between these two units.

8. Device (1) in accordance with one of the preceding claims, wherein the spirometer unit (2, 2a), the activity measurement unit (3) and the evaluation unit (4) can be detachably connected to one another.

9. Device (1) in accordance with one of the preceding claims, wherein the measurement data of the pulmonary respiration of the subject and the measurement data of their physical activity can be compared using the evaluiation unit (4) for a diagnosis.

10. Device (1) in accordance with claim 9, wherein the comparison can be made by means of associated and/or stored time values determined with the timer.

11. Device (1) in accordance with one of the preceding claims, wherein correlated, compared or summarised measurement data or data sets can be output by the evaluation unit (4) via an output unit of the evaluiation unit (4), be stored in a storage unit, or transmitted to an external unit.

12. Device (1) in accordance with one of the preceding claims, wherein the device (1) is portable.

13. Method for determining exacerbation-related parameters with a device (1) in accordance with one of the preceding claims, wherein the method comprises the following steps:
- Measuring a pulmonary function value of the subject with the spirometer unit (2, 2a) at a first time of a first day;
- Measuring a pulmonary function value of the subject with the spirometer unit (2, 2a) at a second time of the first day;
- Measuring a motor movement activity of the arms or legs of the subject over a first period of time with the activity measurement unit (3) up to a third time of the first day and summarising the motor movement activity measured over a period of time of one day as a first summarised value;
- Storing the measurement data of the spirometer unit (2, 2a) and the first summarised value in an evaluation unit (4);
- Calculating corresponding estimated lung function values for recommended times from lung function values measured at differering times of the current day by linearization.

14. Method in accordance with claim 13, wherein the method comprises the additional steps:
- Formation of a difference value (D3) of the lung functrion values for the two recommended times, and
- Assigning a corresponding day to this difference value (D3) as time value.

## Revendications

1. Dispositif (1) pour détecter des paramètres pertinents pour des exacerbations, dans le cadre d'une maladie pulmonaire d'un sujet à examiner, comprenant
- une unité à spiromètre (2, 2a) pour mesurer une fonction pulmonaire du sujet ;
- une unité de mesure d'activité (3) pour mesurer une intensité de mouvement des bras ou des jambes par rapport au tronc du sujet, grâce à une mesure de l'accélération ou de la vitesse de l'unité de mesure d'activité (3), l'unité de mesure d'activité (3) étant conçue pour être portée directement sur le sujet,
- une unité d'analyse (4) pour recueillir des données de mesure de l'unité à spiromètre (2, 2a) et de l'unité de mesure d'activité (3), ladite unité d'analyse (4) étant conçue pour réunir des données de mesure et leur affecter une valeur de temps, une multiplicité de données de mesure mesurées par l'unité de mesure d'activité (3) sur une période d'une journée entière étant réunies à l'aide de l'unité d'analyse (4) en une valeur quotidienne pour l'activité motrice, et l'unité d'analyse (4) étant conçue pour réunir en deux valeurs de fonction pulmonaire des valeurs de mesure de la respiration pulmonaire mesurées en une journée par l'unité à spiromètre (2, 2a), et pour affecter à ces deux valeurs de fonction pulmonaire deux valeurs de temps correspondantes ; et
- une horloge pour détecter, affecter et/ou mettre en mémoire des valeurs de temps correspondant aux données de mesure de l'unité à spiromètre (2, 2a) et de l'unité de mesure d'activité (3),
les deux valeurs de temps correspondantes des valeurs de fonction pulmonaire étant deux heures de la journée conseillées, et l'unité d'analyse (4) étant conçue pour calculer, grâce à une linéarisation, des valeurs de fonction pulmonaire estimées correspondantes pour lesdites heures conseillées, à partir de valeurs de fonction pulmonaire de la journée en cours qui sont déterminées à des heures différentes.

2. Dispositif (1) selon la revendication 1, dans lequel l'unité d'analyse (4) est conçue pour former une valeur différentielle (D3) des valeurs de fonction pulmonaire pour les deux heures de la journée conseillées, et pour affecter à' cette valeur différentielle (D3) comme valeur de temps un jour correspondant.

3. Dispositif (1) selon l'une des revendications précédentes, dans lequel avec l'unité de spiromètre (2, 2a) peuvent être mesurés un volume courant ou pulmonaire, un débit expiratoire, un débit massique et/ou un débit volumétrique de la respiration pulmonaire du sujet à examiner.

4. Dispositif (1) selon l'une des revendications précédentes, dans lequel l'unité à spiromètre (2, 2a) comporte un capteur de volume, un capteur de débit volumique, un capteur de débit massique, un capteur de pression, un capteur de température et/ou un capteur d'humidité.

5. Dispositif (1) selon l'une des revendications précédentes, dans lequel avec l'unité de mesure d'activité (3) peuvent être mesurés une force, une accélération, une durée, une direction, une trajectoire, une vitesse, un moment angulaire et/ou un couple d'un mouvement du sujet.

6. Dispositif (1) selon l'une des revendications précédentes, dans lequel l'unité de mesure d'activité (3) comporte un capteur d'inertie de masse, un gyroscope, un capteur de mouvement, un capteur d'accélération, un capteur de vitesse, un gyroscope laser, un gyroscope à fibre optique ou une unité de détection de position basée sur un GPS ou sur la téléphonie mobile.

7. Dispositif (1) selon l'une des revendications précédentes, dans lequel l'unité à spiromètre (2, 2a), l'unité de mesure d'activité (3) et l'unité d'analyse (4) sont aptes à être reliées par l'intermédiaire d'un réseau de communication à fil ou sans fil, de sorte qu'une transmission des données de mesure entre ces unités est possible.

8. Dispositif (1) selon l'une des revendications précédentes, dans lequel l'unité à spiromètre (2, 2a), l'unité de mesure d'activité (3) et l'unité d'analyse (4) sont aptes à être fixées les unes aux autres de manière amovible.

9. Dispositif (1) selon l'une des revendications précédentes, dans lequel avec l'unité d'analyse (4), les données de mesure de la respiration pulmonaire du sujet et les données de mesure de son activité motrice peuvent être confrontées en vue d'un diagnostic.

10. Dispositif (1) selon la revendication 9, dans lequel la confrontation peut se faire par l'intermédiaire de valeurs de temps détectées, affectées et/ou mises en mémoire avec l'horloge.

11. Dispositif (1) selon l'une des revendications précédentes, dans lequel des données de mesure ou ensembles de données corrélés, comparés ou réunis peuvent être émis par l'unité d'analyse (4) par l'intermédiaire d'une unité d'émission de l'unité d'analyse (4), mis en mémoire dans une unité de mémoire, ou transmis à une unité externe.

12. Dispositif (1) selon l'une des revendications précédentes, ce dispositif (1) étant portatif.

13. Procédé pour détecter des paramètres pertinents pour des exacerbations avec un dispositif (1) selon l'une des revendications précédentes, le procédé comprenant les étapes suivantes :
- la mesure d'une valeur de fonction pulmonaire du sujet avec l'unité à spiromètre (2, 2a) à un premier moment d'un premier jour ;
- la mesure d'une valeur de fonction pulmonaire du sujet avec l'unité à spiromètre (2, 2a) à un deuxième moment du premier jour ;
- la mesure d'une activité motrice des bras ou des jambes du sujet sur une première période, avec l'unité de mesure d'activité (3), jusqu'à un troisième moment du premier jour, et le regroupement de l'activité motrice mesurée sur une journée, pour en faire une première valeur regroupée ;
- la mise en mémoire des données de mesure de l'unité à spiromètre (2, 2a) et de la première valeur regroupée, dans une unité d'analyse (4) ;
- le calcul, grâce à une linéarisation, de valeurs de fonction pulmonaires estimées correspondantes pour des heures de la journée conseillées, à partir de valeurs de fonction pulmonaire de la journée en cours qui sont mesurées à des heures différentes.

14. Procédé selon la revendication 13, le procédé comprenant les étapes supplémentaires suivantes :
- la formation d'une valeur différentielle (D3) des valeurs de fonction pulmonaire pour les deux heures de la journée conseillées ; et
- l'affectation d'une journée correspondante à cette valeur différentielle (D3) comme valeur de temps.
